# FASCICULE DE BREVET EUROPEEN

(11) **EP 0 824 536 B1**
(45) Date de publication et mention de la délivrance du brevet: **22.08.2001**
(21) Numéro de dépôt: 96916196.7
(22) Date de dépôt: 10.05.1996
(51) Int. Cl.: C07F 17/02, C07F 15/02, A61K 31/295

(54) **COMPLEXES ORGANOMETALLIQUES DU FER ANTIPALUDIQUES**
ORGANO-EISENKOMLEXE MIT WIRKUNG GEGEN MALARIA
ANTIMALARIAL ORGANOMETALLIC IRON COMPLEXES

(30) Priorité: 10.05.1995 FR 9505532
(43) Date de publication de la demande: 25.02.1998
(73) Titulaire: UNIVERSITE DES SCIENCES ET TECHNOLOGIES DE LILLE, 59650 Villeneuve-d'Ascq (FR)
(72) Inventeur: BROCARD, Jacques, F-59155 Faches-Thumesnil (FR); LEBIBI, Jacques, Franceville (GA); MACIEJEWSKI, Lucien, F-59655 Villeneuve-d'Ascq (FR)
(74) Mandataire: Vaillant, Jeanne
(86) Numéro de dépôt international: FR9600721
(87) Numéro de publication internationale: WO9635698

(56) Documents cités:
- EP-A- 0 004 894
- GB-A- 1 470 210
- CHEMICAL ABSTRACTS, vol. 119, no. 13, 27 Septembre 1993 Columbus, Ohio, US; abstract no. 131035p, ONG, C.W. ET AL.: "A FERROCENE-INTERCALATOR CONJUGATE WITH A POTENT CYTOTOXICITY" XP002009648 & BIOORG. MED. CHEM. LETT., vol. 2, no. 9, 1992, pages 929-932,
- CHEMICAL ABSTRACTS, vol. 122, no. 3, 16 Janvier 1995 Columbus, Ohio, US; abstract no. 31669s, KLIMOVA, Y. ET AL.: "BIOLOGICAL ACTIVITY OF FERROCENYL-SUBSTITUTED DERIVATIVES OD .DELTA.4-TETRAHYDROPHTHALIC ACID" XP002009649 & KHIM.-FARM. ZH., vol. 28, no. 6, 1994, pages 30-32,

## Description

L'invention concerne des complexes organométalliques du fer présentant des propriétés particulièrement intéressantes à titre d'agents antipaludiques. L'invention concerne notamment l'insertion de groupements ferrocènes au sein de la structure de molécules possédant des propriétés antipaludiques.

Depuis plusieurs années, le paludisme continue d'être une cause de mortalité majeure dans plusieurs pays en voie de développement. L'effritement de l'arsenal chimio-thérapique lié au développement de la résistance des souches est un des principaux problèmes rencontrés dans la lutte antipaludique. La chloroquinine est l'antipaludéen le plus utilisé dans le monde et des souches chloroquinorésistantes sont apparues dans toutes les zones impaludées. Il est donc envisageable qu'à terme les difficultés rencontrées dans le traitement d'infections liées au paludisme seront de plus en plus importantes. Bien que des ressources considérables soient consacrées à la lutte antipaludique, peu d'alternatives aux médicaments présentement utilisés ont été proposées.

Les inventeurs ont découvert qu'en associant à des molécules de structure proche de celle d'antipaludéens un ou plusieurs atomes de fer, par exemple par l'entremise de l'insertion d'un groupement ferrocène à l'intérieur d'analogues de substances telle la quinine, la chloroquinine, la mépacrine et la primaquine, on augmentait de façon substantielle l'activité thérapeutique de ces substances. Les inventeurs ont également découvert que l'utilisation des composés de l'invention permet d'éviter les rechutes à long terme, souvent associés aux traitements antipaludiques conventionnels.

La grande vitesse de reproduction du plasmodium falcioarum et son utilisation du fer ont conduit à des tentatives de destruction par privation de fer dans l'utilisation d'hydroxyamates de ferrioxamine. Bien que ces essais aient donné des résultats satisfaisants, les inventeurs ont orienté leur stratégie sur une démarche inverse. Il s'agit plutôt d'utiliser l'affinité du plasmodium pour le fer pour augmenter la probabilité de rencontre avec la molécule antipaludique.

L'utilisation des complexes organo-métalliques demeure jusqu'à présent marginale en chimie thérapeutique. Le ferrocène en particulier n'apparaît que dans des analogues des prostaglandines, le Ferroceron (médicament utilisé pour combattre les déficiences en fer) et des analogues de l'hexestrol.

Le document CA, vol. 119, n° 13 (1993) n° 131035p ONG, C.W. et al., "Ferrocene-intercalator conjugate with a potent cytotoxicity" décrit la synthèse d'un composé conjugué ferrocène-acrydine et ses propriétés cytotoxiques potentielles.

Le document CA, vol. 22, n° 3 (1995) n° 31669s KLIMOVA, Y. et al., "Biological activity of ferrocenyl-substituted derivatives of Δ⁴-tetrahydrophthalic acid" décrit la synthèse de dérivés de l'acide Δ⁴-tétrahydrophthalique comportant un ferrocène, et l'activité anti-inflammatoire de certains de leurs sels.

GB-A-1 470 210 décrit des pénicillines semi-synthétiques associées au ferrocène et leurs propriétés antibiotiques.

EP-A-0 004 894 décrit des compositions phytosanitaires comportant du 3,5,5-triméthoxylhexanoyl-ferrocène pour le traitement de plantes carencées en fer.

Aucun de ces documents ne décrit des complexes organométalliques antipaludiques.

L'invention concerne donc des complexes organométalliques du fer comprenant au moins un des éléments structurels caractéristiques d'une molécule possédant des propriétés antipaludiques ainsi qu'un ou plusieurs atomes de fer. La référence à des éléments structurels caractéristiques d'une molécule possédant des propriétés antipaludiques est utilisée pour désigner certaines portions caractéristiques de molécules antipaludiques qui confèrent à ces molécules leurs propriétés.

Des éléments structurels caractéristiques de molécules possédant des propriétés antipaludiques sont variés et peuvent être facilement reconnus par la personne versée dans l'art. On notera cependant la nécessité d'utiliser la structure de base déterminant l'activité antipaludique. Par exemple, dans le cas des quinines, la présence d'un substituant amino alcool sur la quinoléine est essentiel, de même qu'un substituant diamine sur la quinoléine est essentiel pour les molécules modifiées de type chloroquinine, de même qu'un substituant est essentiel pour les molécules modifiées de type mépacrine. Pour ces molécules, les quinoléines ou les acridines sont considérées comme des éléments structurels de base conférant les propriétés antipaludiques désirées.

Il s'agissait donc de reproduire aussi fidèlement que possible la structure de base de ces molécules tout en y attachant, et de façon préférée en y insérant, des substituants permettant de conférer à ces molécules un caractère organométallique. L'invention est donc basée sur l'association d'un atome de fer et d'un ligand organique proche de molécules dont les propriétés antipaludiques sont connues. De façon préférée, le fer est apporté par le ferrocène dont la chimie est connue et plusieurs dérivés sont commercialisés.

Il est important de souligner ici que les inventeurs n'ont pas cherché uniquement à greffer une molécule contenant des atomes de fer (le ferrocène par exemple) à des composés antipaludiques existants. Des impératifs de synthèse organique auraient pu rendre ce type d'opération difficile, mais en tout état de cause, les inventeurs cherchaient plutôt à positionner les atomes de fer de façon à les rendre aussi facilement accessibles que possible aux souches de Plasmodium Falciparum visées.

En fait, les inventeurs ont cherché à remplacer une partie hydrophobe par un groupement ferrocène par exemple, et à placer ce groupement ferrocène à l'intérieur de la molécule de façon à perturber le moins possible la géométrie globale de l'ensemble. Le choix de la position du ferrocène est donc fonction d'un endroit sur la molécule où les altérations géométriques modifient le moins possible les effets structurels globaux de la molécule.

Selon l'une des réalisations préférentielles, les complexes organométalliques du fer de l'invention comprennent au moins un des éléments structurels de base suivants : 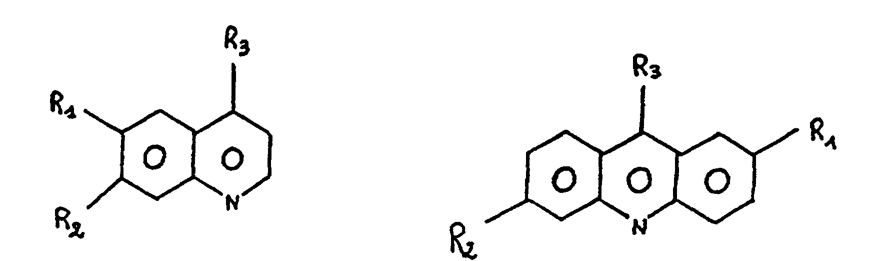 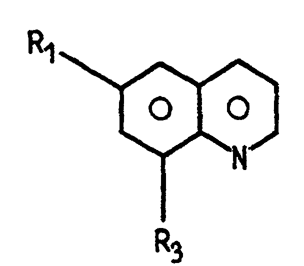
dans lesquels R1 est hydrogène, alkyl, alkoxy, acyl, halogène ou étheralkyl aldéhyde, hydroxy, hydroxyalkyl, alkoxyalkyl, acyloxyalkyl, acétylalkyl, de préférence hydrogène ou éthermethyl;
R2 est hydrogène, alkyl, alkoxy, halogène, acyl ou étheralkyl aldéhyde, hydroxy, hydroxyalkyl, alkoxyalkyl, acyloxyalkyl, acétylalkyl, de préférence hydrogène ou halogène ; et
R3 est un groupe amine ou hydroxyalkyle relié à un groupe ferrocényle par l'entremise d'une liaison covalente ;
ainsi que les sels de ces composés, par exemple les sels de ces composés, par exemple les sels tartrate ou di-tartrate.

De préférence, lorsque le terme alkyl est utilisé dans le contexte de la présente invention, on fait référence à un alkyl ayant entre 1 et 10, de façon préférée entre 1 et 4 atomes de carbone.

Bien que le ferrocène semble être le substituant de choix pour conférer un caractère organométallique aux composés de l'invention, on peut envisager l'utilisation d'autres substituants qui rendraient possible la présentation d'un ou de plusieurs atomes de fer susceptibles d'être absorbés par la souche de plasmodium infectante. On peut donc envisager entre autres l'utilisation de substituants tels les diène fer-tricarbonyl.

Selon une autre réalisation préférentielle de l'invention, le complexe organométallique du fer est caractérisé en ce qu'il possède la structure suivante : 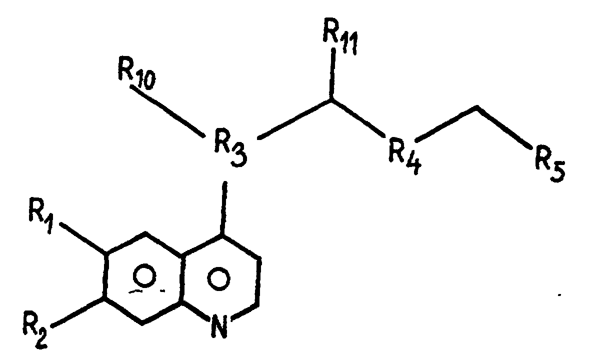 dans laquelle
R1, R2 et R3 sont tels que définis précédemment ;
R4 est un groupement alkyle, alkoxy, acyl, hydroxyalkyl, acyloxyalkyl, acétylalkyl ou ferrocényle ;
R5 est un groupement amine éventuellement substitué par un ou plusieurs groupement alkyl, particulièrement méthyl ou par un ou plusieurs groupes alkyl-ferrocényle ;
R10 est hydrogène, alkyle ou un groupe alkyl-ferrocényle; et
R11 est hydrogène ou alkyle.

Parmi les composés particulièrement intéressants, l'utilisation des composés ayant la structure suivante est envisagée : 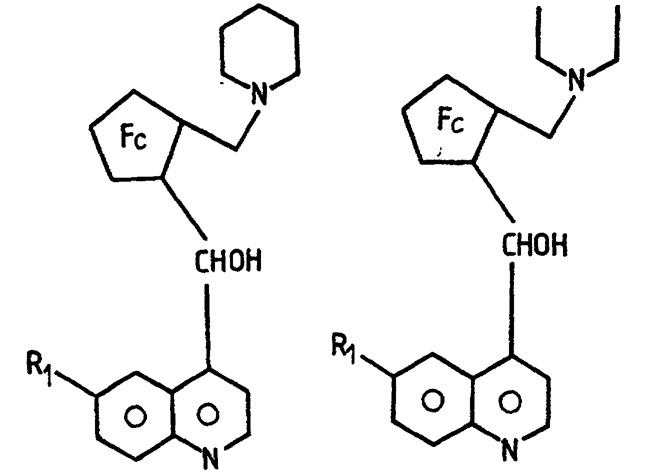 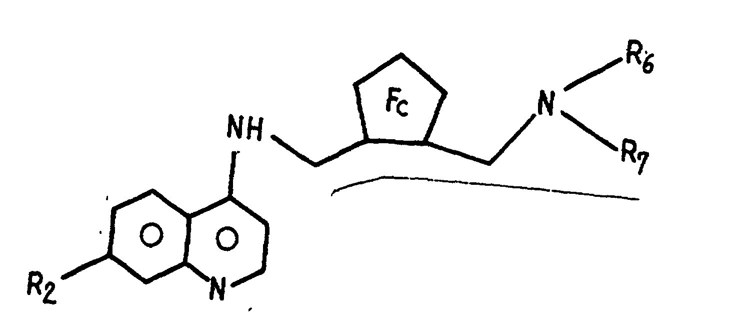 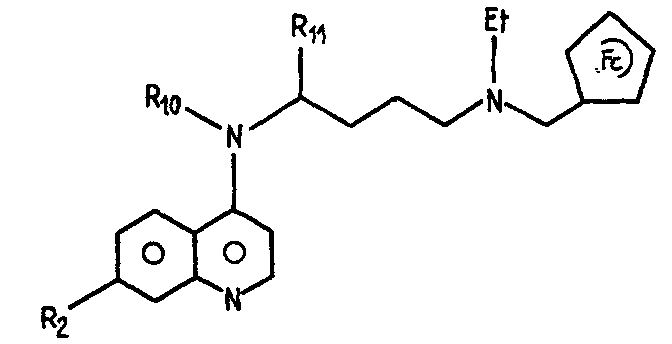 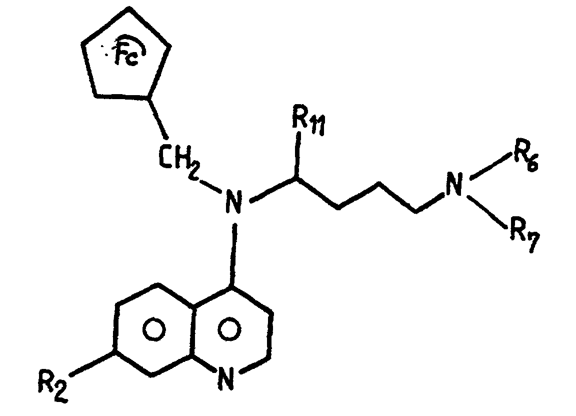 dans lesquels
R1, R2, R10 et R11 sont tels que définis précédemment ; et R6 et R7 sont identiques ou différents et sont choisis parmi hydrogène, alkyl-ferrocène, ferrocène, alkyl, particulièrement méthyl ou éthyl, mais également le groupement 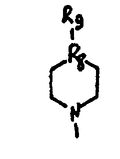 où R8 est un -CH- ou un hétéroatome, et R9 est hydrogène, alkyl, alkoxy, acyl, halogène ou étheralkyl aldéhyde, hydroxy, hydroxyalkyl, alkoxyalkyl, acyloxyalkyl, acetylalkyl, pour former de préférence les groupes pipéridino 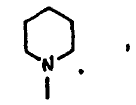 morpholino 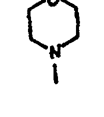 ou piperazino 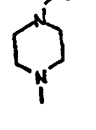 Le groupement 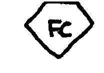 représente un substituant ferrocène tel que connu par la personne versée dans l'art.

L'invention concerne également les composés comprenant un groupe acridine et possédant la structure suivante : 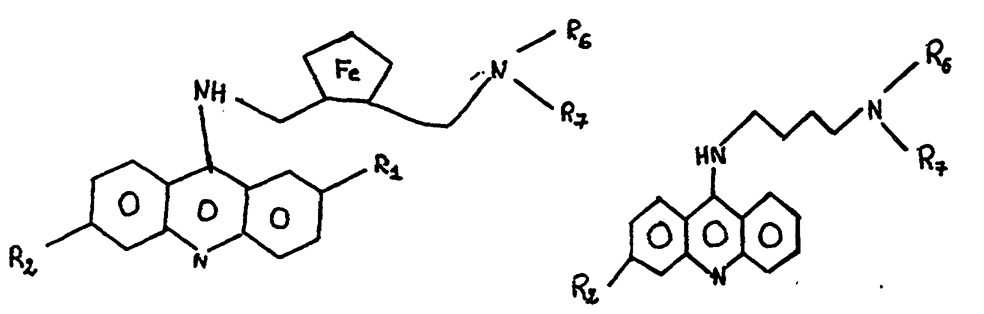 dans laquelle R1, R2, R6 et R7 sont tels que définis précédemment.

De plus, l'invention concerne des complexes organométalliques pouvant être considérés comme des analogues de la primaquine ayant la structure suivante : 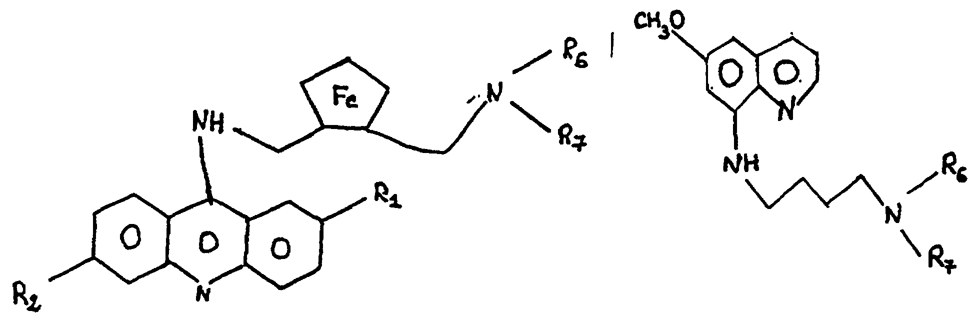 dans laquelle R6 et R7 sont tels que définis précédemment.

L'invention concerne également des compositions pharmaceutiques comprenant un des composés décrits précédemment en association avec un excipient pharmaceutique acceptable ainsi qu'une méthode pour le traitement du paludisme qui consiste à administrer à un patient une dose thérapeutique d'un des composés mentionnés précédemment.

Des tests in vitro et in vivo effectués pour déterminer l'activité des complexes organométalliques de l'invention sur des souches de laboratoire et des souches de terrain ont été effectués. Les tests in vitro. qui sont basés sur l'assimilation d'hypoxanthine radioactive par des parasites ont démontré que les complexes organométalliques de l'invention ont, de façon générale, une action plus importante sur l'inhibition de la parasitémie que les composés normalement utilisés telle la chloroquinine ou la quinine. De plus, des tests ont démontré une action marquée sur une souche chloroquino-résistante.

### Préparation de certaines réalisations préférentielles des complexes organométalliques du fer de l'invention

La synthèse des complexes organométalliques de l'invention fait appel aux méthodes classiques de la chimie organométallique, soit la réaction de Mannich, la lithiation et la condensation sur un aldéhyde. Par exemple, le passage du ferrocène au dialkylaminométhylferrocène par réaction du méthanal et de la dialkylamine (ou la pipéridine ou du di(dialkylamino)méthane est connu. Il s'agit de la réaction de Mannich. Le choix de l'amine permet de moduler les chaînes alkyles portées par l'azote dans le produit final. La lithiation est décrite dans la littérature également. La condensation sur la 4-formyl quinoléine conduit au composé désiré. Suivant cette méthode et en se basant sur certaines molécules modèles, les molécules suivantes sont synthétisées :
1) Molécule modèle = Quinine synthèse 1 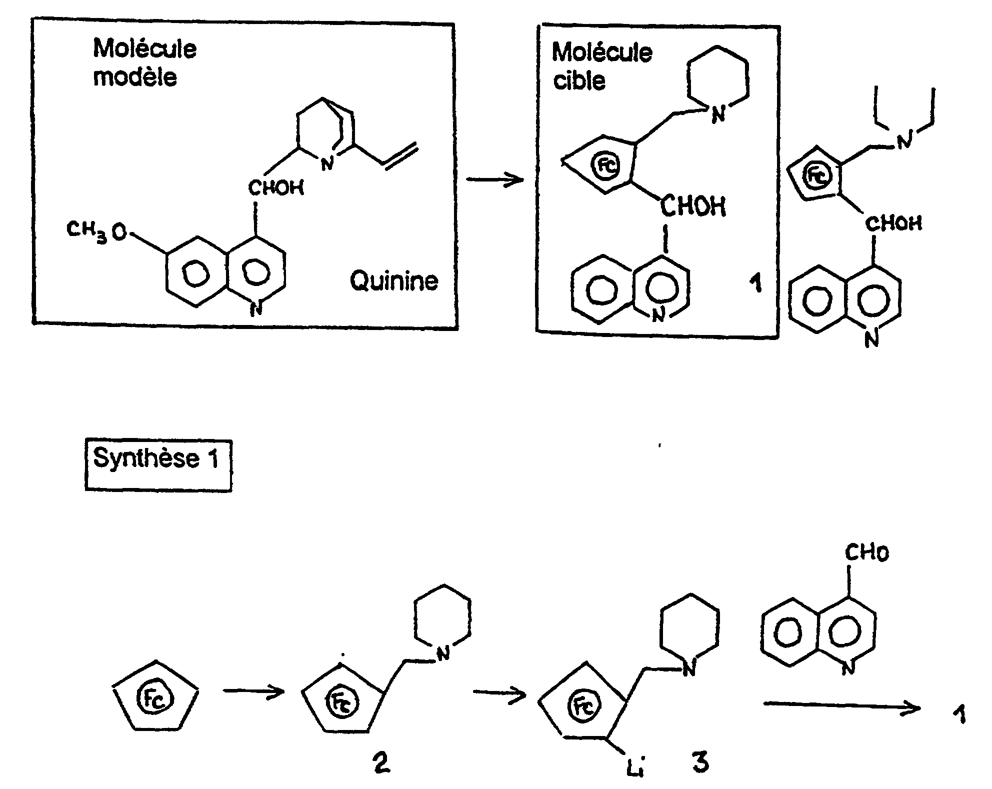
   Le passage du ferrocène au dialkylamino méthyl ferrocène 2 par action du méthanal et d'une dialkyl amine (ou la pipéridine) ou du di(dialkylamino)méthane est connu (réaction de MANNICH). Le choix de l'amine permet de moduler les chaînes alkyles portées par l'azote dans le produit final. La lithiation de 2 est décrite dans la littérature. La condensation de 3 sur la 4-formyl quinoléine conduira à 1. L'étude stéréochimique de ce produit qui porte deux centres de chiralité sera effectuée.
2) Molécule modèle = Chloroguinine Synthèses 2 et 3 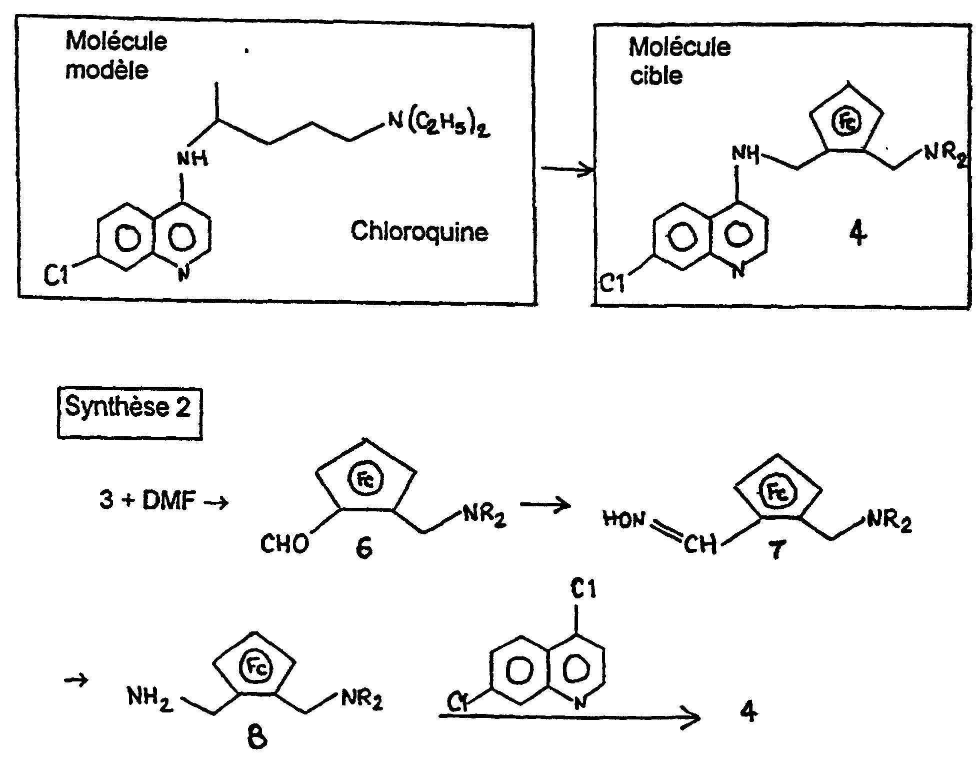
   La condensation de 3 sur la diméthylformamide conduit à l'aldéhyde 6. L'oximation et la réduction de 6 donnent 7 puis 8. La condensation de 8 sur la 4-7 dichloroquinoléine aboutit au composé 4. 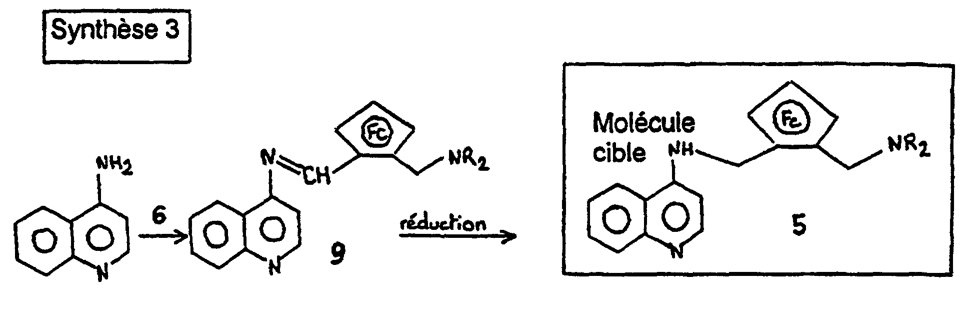
   L'aldéhyde 6 est condensé avec la 4-aminoquinoléine. L'imine 9 formée sera hydrogénée en 5, homologue non chloré de 4.
3) Molécule modèle = Mépacrine synthèses 4 et 5 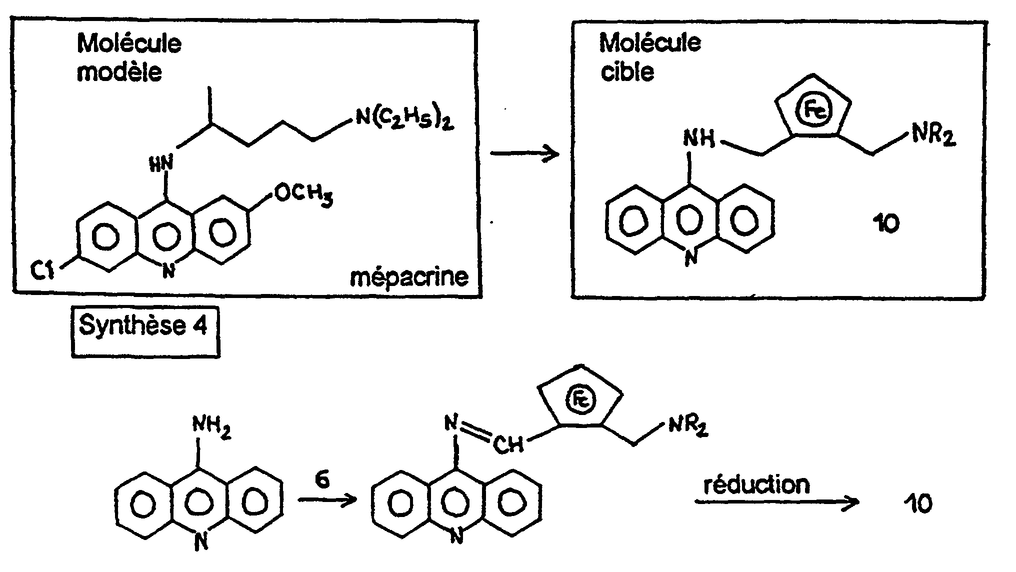
   L'aldéhyde D est condensé avec la 6-aminoacridine, qui est commerciale. L'imine formée sera hydrogénée en 10, homologue non chloré et non méthoxylé de la mépacrine. 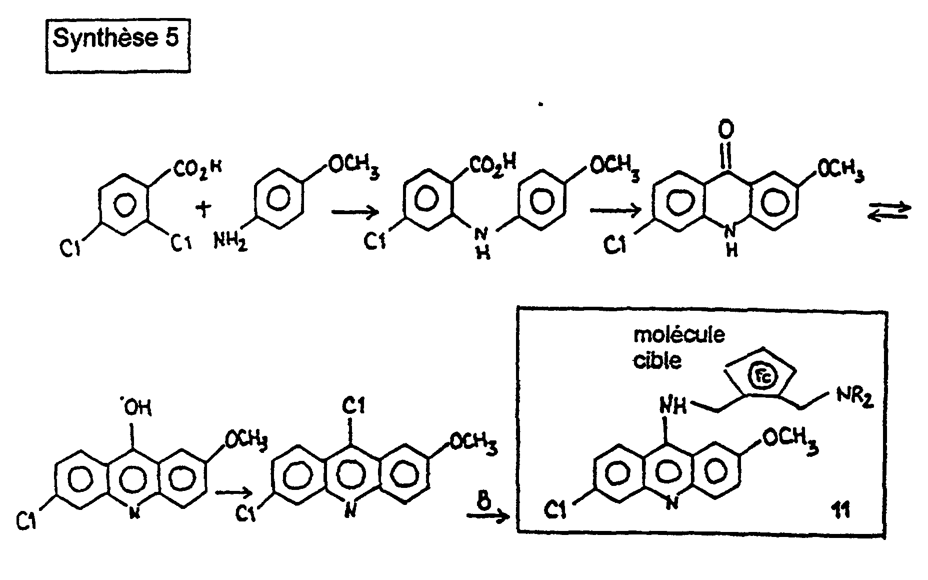
   Cette synthèse, connue jusqu'au composé dichloré, permet l'introduction des substituants présents sur la molécule cible.
4) Molécule modèle = Primaquine synthèses 6,7,8 et 9

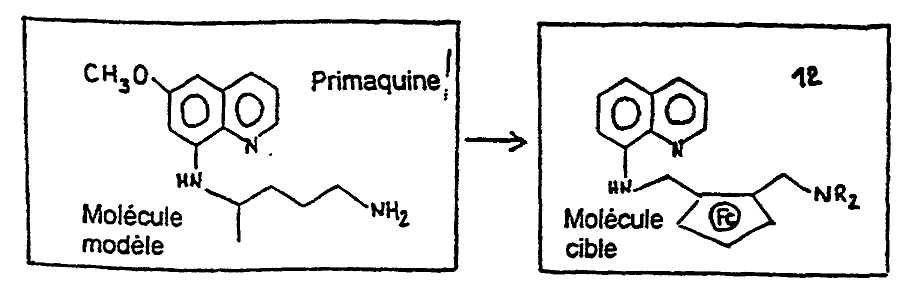

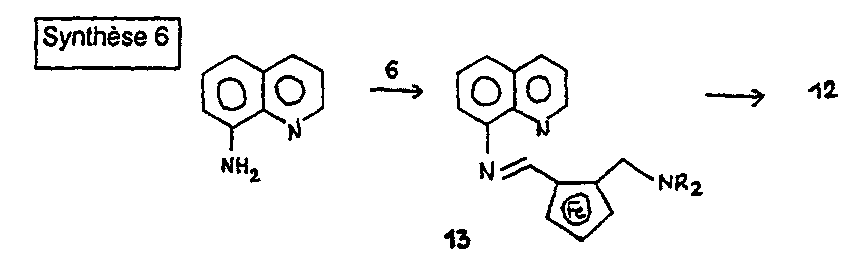

L'aldéhyde 6 est condensé sur la 8-aminoquinoléine et l'imine 13 est hydrogénée en 12.

L'homologue de 12 méthoxylé en 6 s'obtient à partir de la 6-méthoxy 8-amino quinoléine dont la préparation est connue (synthèse de Skraup) :

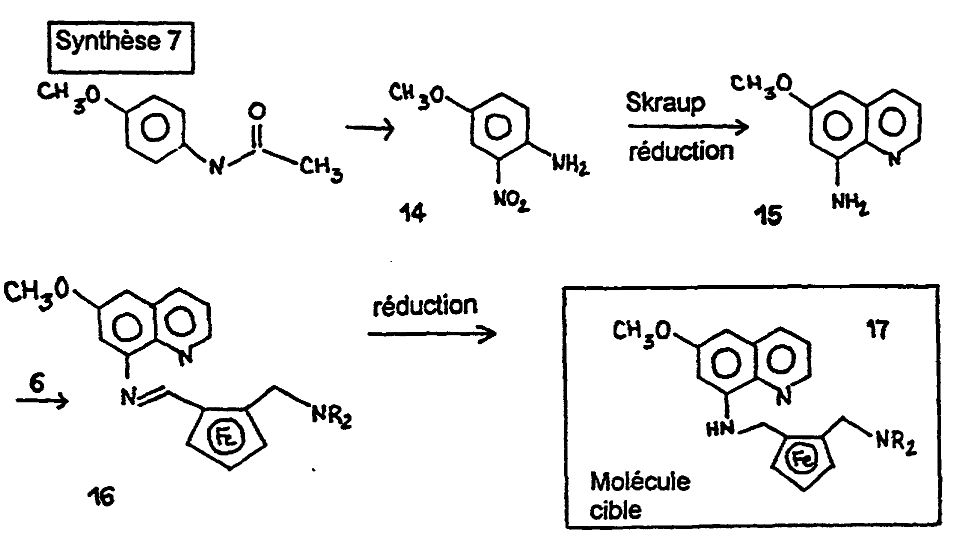

L'homologue méthylé en α de l'azote secondaire est obtenu par action de 3 sur l'acétonitrile :

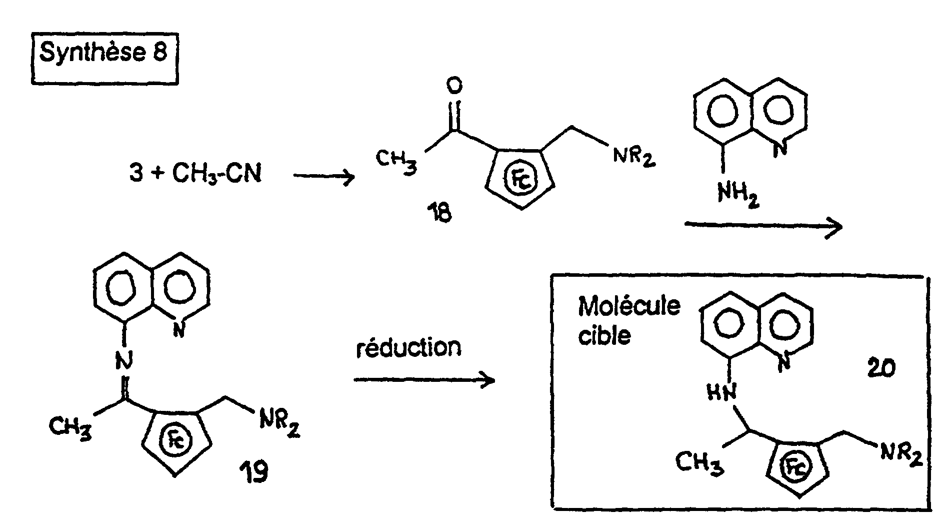

L'homologue méthylé en α de l'azote secondaire et méthoxylé en 6, comme l'est la Primaquine, est obtenu par action de 18 sur 15 :

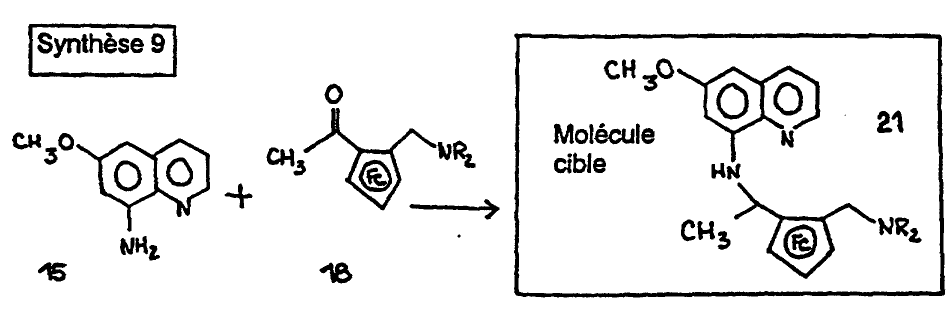

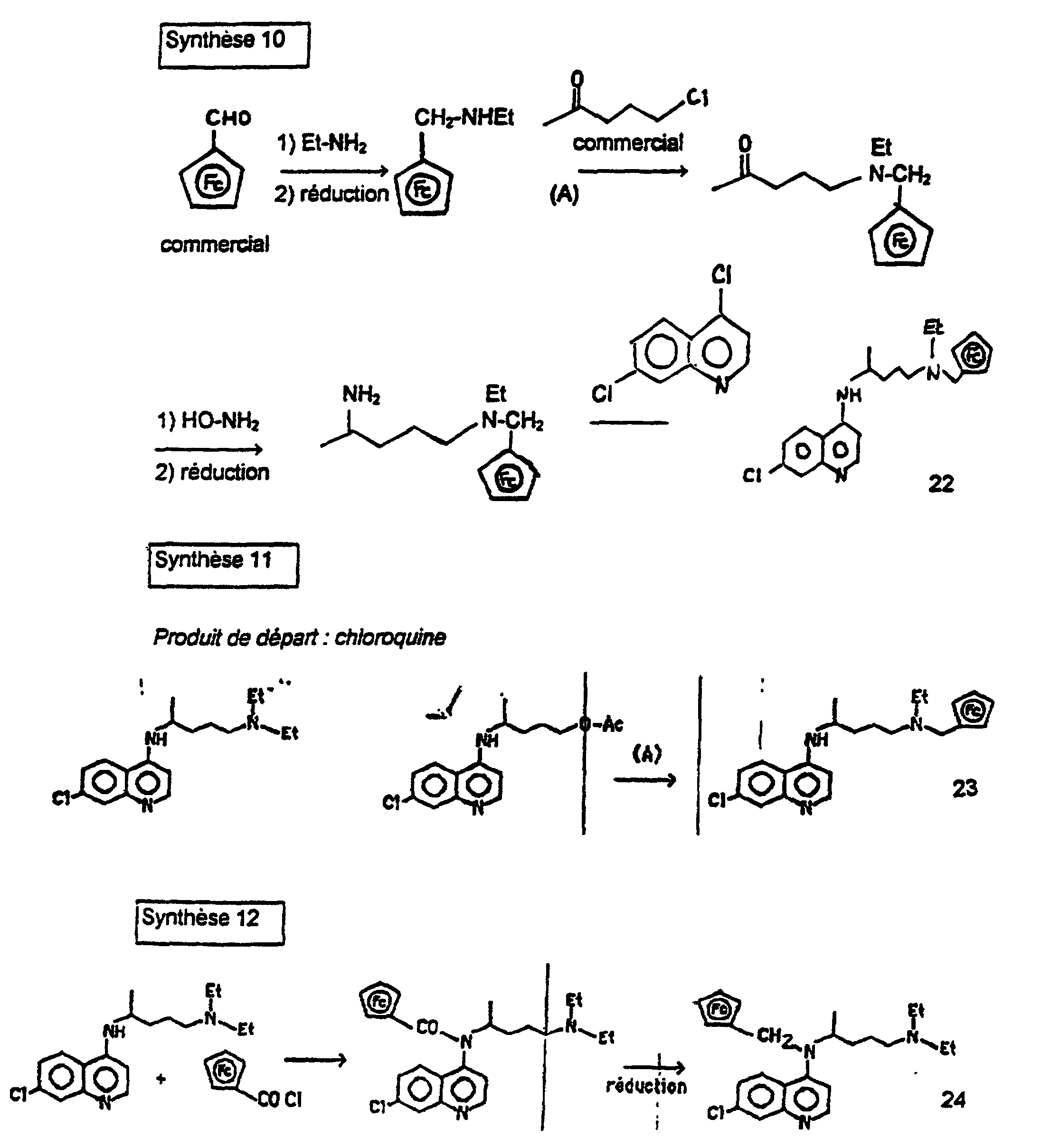

Ces 12 synthèses conduisent à 12 séries de molécules obtenues en utilisant, pour la réaction de MANNICH de départ, des amines diversement substituées. Les sels de ces composés (par exemple les sels tartrate ou di-tartrate) peuvent être facilement obtenus par la personne versée dans l'art.

### BIBLIOGRAPHIE

1) K DOMBROWSKI, W. BALDWIN et J. SHEATS, J. Organomet. Chem., 1986, 281.
2) A. RYABOV, Angew. Chem. Int. Ed. Engl., 1991, 931.
3) G. JAOUEN et A. VESSIERES, Pure Appli. Chem. 1989, 61, 56; G. JAOUEN, A. VESSIERES, et I. Butler, Acc. Chem. Res. 1993, 26, 361 ; id. Organomet., 1993, 12, 4545
4) M. GRUSELLE, B. MALESIEUX, L. TROITSKAYA, V. SOKOLOV, L. EPSTEIN, Y. SHUBRINA, Organomet., 1994, 13, 200.
5) V. SOKOLOV, L. TROISKAYA, N. KRUSKCHOVA, Izv. Acad, Nauk. SSSR, Ser. Kim., 1987, 10, 2387.
6) KOPF et Coll., Angew. Chem., 1984, 96, 446, id. Chem. Hev., 1987, 87, 1137.

### Tests in vitro de complexes organométalliques de l'invention

### a) Souches de terrain

Tel que mentionné précédemment, l'activité in vitro de complexes organométalliques de l'invention a été mesurée sur des souches de terrain. Les composés testés sont les composés SN1, qui correspondent au composé 4 dans lequel -NR₂ est -N(CH₃)₂, SN₂ qui correspond au composé 4 dans lequel -NR₂ est pipéridine et OD3, qui correspond au composé 11 dans lequel -NR'₂ est pipéridine.

La prolifération des parasites est mesurée par incorporation de ³[H] hypoxanthine appliquée aux cultures 16 heures avant la fin de la culture. L'hypoxanthine radioactive est absorbée par les parasites en développement. La quantité absorbée est mesurée par un compteur Beta en coups par minute. L'analyse est généralement faite par comparaison des C.1.50 (concentration inhibant 50% de la prolifération) calculées par interpolation linéaire.

Les résultats présentés dans le tableau 1 ci-dessous démontrent que l'analogue SN1 de la chloroquinine a, d'une manière générale, une action plus importante sur l'inhibition de la parasitémie que la quinine et une action marquée sur la seule souche chloroquinorésistante testée jusqu'à présent

Elle a permis également de constater la forte chloroquinorésistance de ces souches Francevilloises (Cl 50 entre 190 et <1000 nm pour les 7 souches testées).

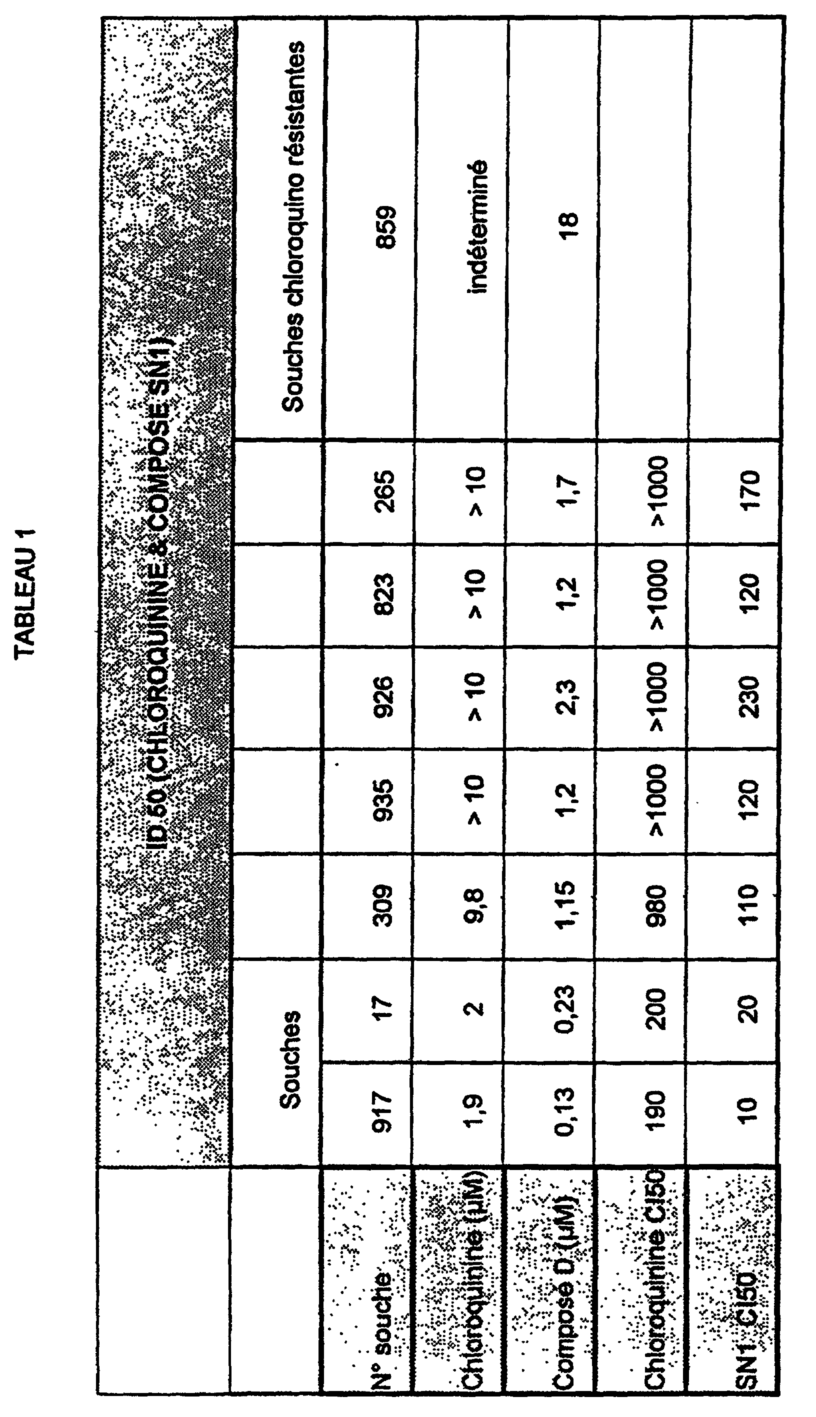

### b) souches chloroquinorésistantes

Trois lignées de *P*. *faciparum* M25 chloroquinosensible et; FCM 6 et FCM 17, chloroquinorésistantes, ont été mises en culture à un taux de parasitémie avoisinant 1%, au stade trophozoïte, pour une durée de 48 heures. Les cultures ont été traitées par les antimalariques (chloroquinine et analogues ferrocène SN1, SN2 et OB3) aux concentrations de 10⁻³, 10⁻², 10⁻¹, 1 et 10 µg/ml en comparaison avec un contrôle (solvant des antimalariques) et des cultures non-traités. Les résultats obtenus avec les lignées révèlent une inhibition par les composés ferrocène de la résistance des souches FCM 6 et FCM 7 à la chloroquinine, variable selon les fonctions terminales rattachées au groupement ferrocène (Tableau 2). Les analogues restent efficaces sur la lignée chloroquino-sensible M25. Il est à noter une différence de sensibilité du composé SN2 entre les lignées FCM 6 et FCM 17, (ce qui laisserait supposer une différence de métabolisme de la molécule entre ces deux souches).

### Tableau 3: C.I.50 (en µg/ml) de la chloroquinine (chloro.) et des analogues ferrocènes (SN1, SN2, SN3) sur des lignées établies de P. falciparum chloroquinine-sensibles (M 25) et chloroquinine-résistantes (FCM 6 et FCM 17).

### Résultats de deux expériences.

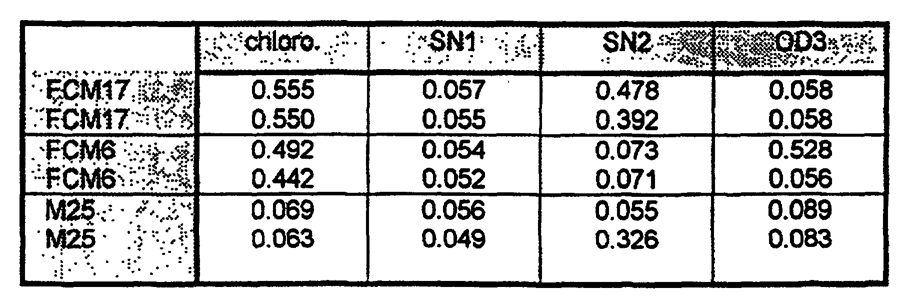

L'introduction d'un groupement ferrocène dans la molécule conserve donc l'efficacité de la chloroquinine chez les souches chloroquinorésistantes. Les inventeurs croient que l'affinité de l'hémoglobine pour le fer favorise l'absorption, ou bien limite le relargage de la chloroquinine qui est particulièrement important chez les souches chloroquinorésistantes. D'autres part, l'avidité des plasmodies vis-à-vis du fer circulant peut également expliquer le rendement des composés ferrocène, surtout si le parasite est incapable de métaboliser le ferrocène.

### Tests de l'activité in vivo d'un complexe organométallique du fer de l'invention présentant une structure analogue à la chloroguinine

A partir des résultats obtenus dans le cadre d'études in vitro, les inventeurs ont sélectionné le composé SN1 pour effectuer des études in vivo. En vue de faciliter l'assimilation du produit par l'organisme en absorption per os, un sel de ce composé, le SN1 di-tartrate, a également été testé in vitro sur des souches chloroquinorésistantes: Les résultats obtenus indiquent une meilleure activité du composé SN1 di-tartrate (SN1-tar) que celle du produit SN1.

Méthodologie: Les souris de laboratoires peuvent être infectées par différentes souches de Plasmodium de rongeur. Des souris SWISS femelles (environ 30gm) (5 par groupe) ont été inoculées à JO par 107 parasites de *P. berghei,* souche létale, chloroquinosensible. Les souris ont été traitées entre JO et J2 par 4 injections sous cutanées de chloroquinine ou de composé SN1, avec une dose totale de 1 ou 10 mg/Kg de poids. La parasitémie et mortalité ont été suivies à partir de J3, pendant au moins 40 jours.
Le composé SN1 et son sel, le SN1 di-tartrate ont été testés.

### Résultats:

Test du composé SN1 et SN3-tar sur *P*. *berghei*, souche N: le résultat de deux expériences est donné dans le tableau suivant:

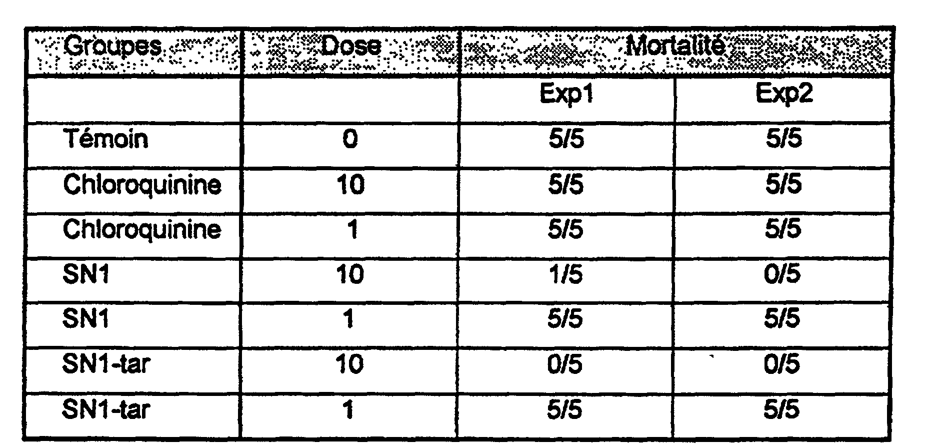

Les composés SN1 et SN1- di-tartrate ont donc une meilleure activité que la chloroquinine in vivo sur les souches utilisées.

D'autre part, des expériences utilisant un Plasmodium de rongeur chloroquinorésistant, P. *yoelii*, souche NS, et le SN1 di-tartrate ont également été effectuées : aucun parasite n'a pu être détecté chez les souris traitées avec 10 mg/Kg de SN1 di-tartrate, alors que toutes les souris traitées par une même dose de chloroquinine présentent une parasitémie importante.

Le composé SN1 di-tartrate est donc actif *in vivo* sur une souche de *P. yoelii* présentant le même type de chloroquinorésistante que *P. faciparum.* Les résultats démontrent également une nette diminution du nombre de rechutes lorsque les composés de l'invention sont utilisés.

Les inventeurs ont également testé *in vivo* avec *P. yoelii* le composé 23 sous forme de sel di-tartrate. Les résultats préliminaires obtenus montrent que ce composé possède une activité équivalente à l'activité de SN1-tar (sel tartrate de SN1).

## Revendications

1. Complexe organométallique du fer comprenant au moins un des éléments structurels de base caractéristiques d'une molécule possédant des propriétés antipaludiques ainsi qu'un ou plusieurs groupes ferrocényle, l'élément structurel caractéristique d'une molécule possédant des propriétés antipaludiques incluant : 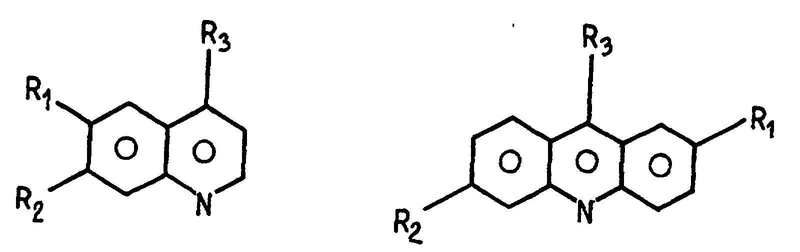 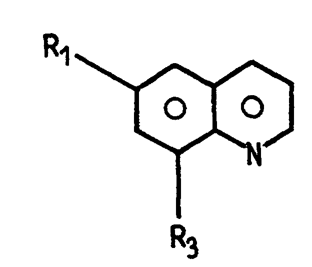 dans lesquelles :
R1 est hydrogène, alkyl, alkoxy, acyl, halogène ou étheralkyl aldéhyde, hydroxy, hydroxyalkyl, alkoxyalkyl, acyloxyalkyl, acétylalkyl, de préférence hydrogène ou étherméthyl;
R2 est hydrogène, alkyl, alkoxy, halogène, acyl ou étheralkyl aldéhyde, hydroxy, hydroxyalkyl, alkoxyalkyl, acyloxyalkyl, acétylalkyl, de préférence hydrogène ou halogène ; et
R3 est un groupement amine ou hydroxyalkyl relié à un groupe ferrocényle par l'entremise d'une liaison covalente et les sels desdits complexes.

2. Complexe selon la revendication 1, caractérisé en ce qu'il possède une des structures suivantes : 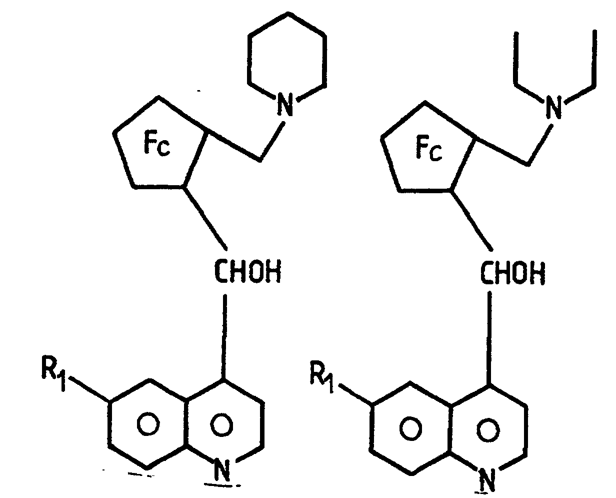 dans lesquelles
R1 est tel que défini précédemment.

3. Complexe selon la revendication 1, caractérisé en ce qu'il possède la structure suivante : 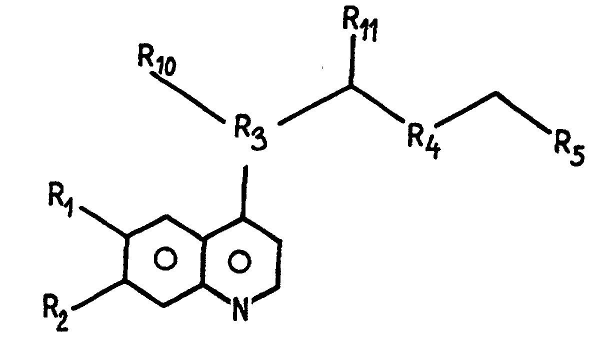 dans laquelle
R1, R2 et R3 sont tels que définis à la revendication 1 ;
R4 est un groupement alkyle, alkoxy, acyl, hydroxyalkyl, acyloxyalkyl, acétylalkyl ou ferrocényle ;
R5 est un groupement amine éventuellement substitué par un ou plusieurs groupement alkyl ou par un ou plusieurs groupes alkylferrocényle;
R10 est hydrogène, alkyle ou un groupe alkyl-ferrocényle; et
R11 est hydrogène ou alkyle.

4. Complexe selon la revendication 3, caractérisé en ce qu'il possède une des structures suivantes: 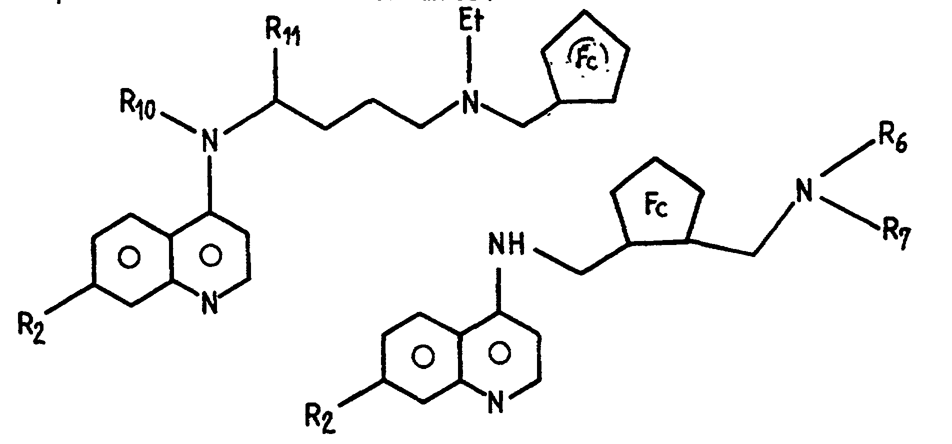 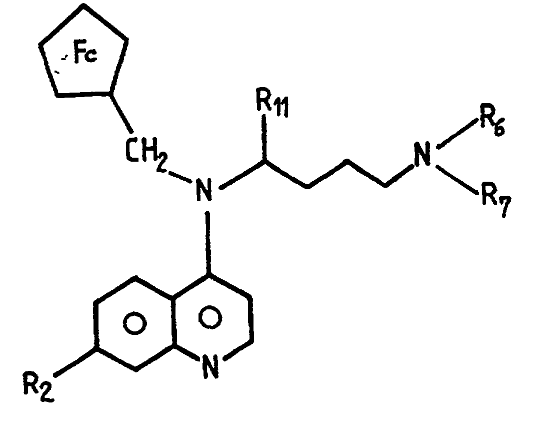 dans lesquelles:
R2, R10 et R11 sont tels que définis précédemment ; et R6 et R7 sont identiques ou différents et sont choisis parmi hydrogène, alkyl-ferrocényle, ferrocényle, alkyl, particulièrement méthyl ou éthyl, mais également le groupement 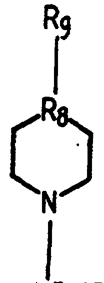 où R8 est un -CH₂- ou un hétéroatome, et R9 est hydrogène, alkyl, alkoxy, acyl, halogène ou étheralkyl aldéhyde, hydroxy, hydroxyalkyl, alkoxyalkyl, acyloxyalkyl, acétylalkyl, pour former de préférence les groupes pipéridino 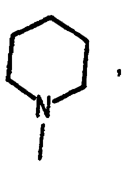 norpholino 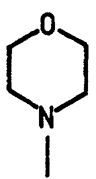 ou piperazine

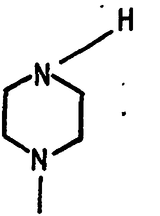

5. Complexe selon la revendication 1, caractérisé en ce qu'il possède la structure suivante: 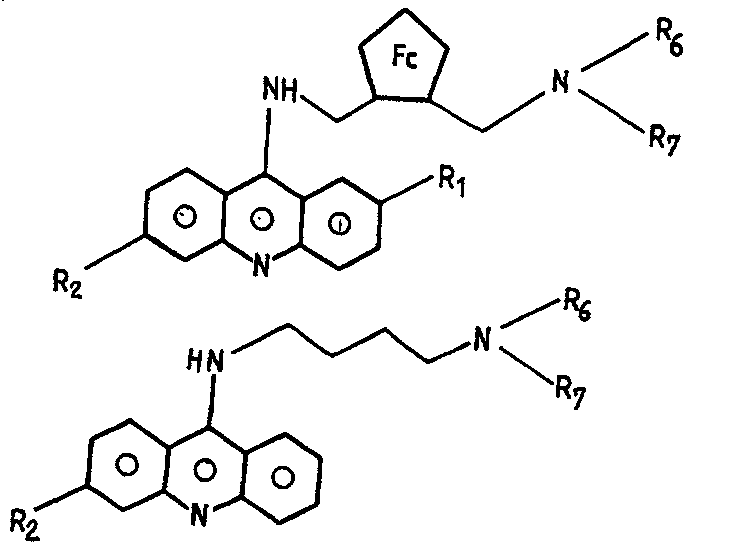 dans laquelle
R1 et R2 sont tels que définis à la revendication 1 ; et
R6 et R7 sont tels que définis à la revendication 4.

6. Complexe selon la revendication 1, caractérisé en ce qu'il possède la structure suivante : 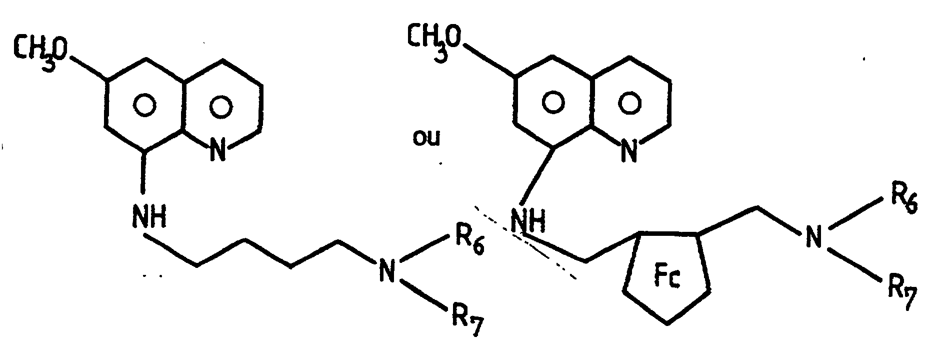 dans laquelle
R6 et R7 sont tels que définis à la revendication 4.

7. Complexe selon l'une des revendications 1 à 6, caractérisé en ce que les sels sont des sels tartrate ou di-tartrate.

8. Composition pour le traitement du paludisme, caractérisée en ce qu'elle comprend un complexe organométallique tel que défini aux revendications 1 à 7, en combinaison avec un excipient pharmaceutique acceptable.

9. Utilisation d'un composé selon l'une quelconque des revendications 1 à 7 pour la préparation d'un médicament destiné au traitement du paludisme.

## Patentansprüche

1. Organo-Eisenkomplex umfassend wenigstens eines von Grundstrukturelementen, welche für ein Molekül charakteristisch sind, das Anti-Malariaeigenschaften besitzt, sowie eine oder mehrere Ferrocengruppen, wobei das Strukturelement, das charakteristisch für ein Molekül mit Anti-Malariaeigenschaften ist 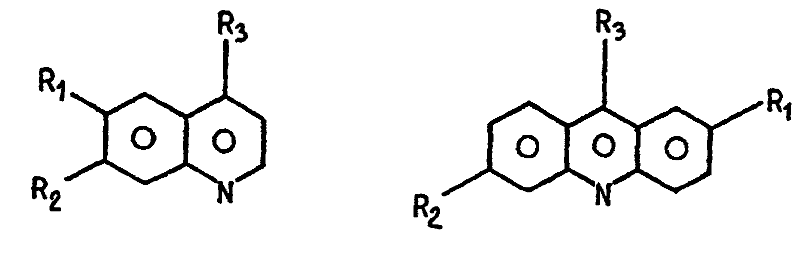 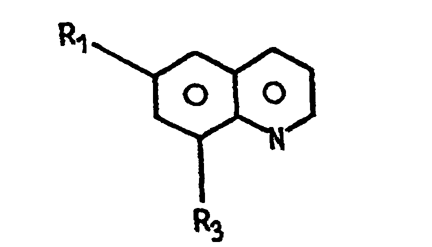 umfasst, worin:
R1 Wasserstoff, ein Alkyl-, Alkoxy-, Acyl-, Halogen- oder Etheralkylaldehyd-, Hydroxy-, Hydroxyalkyl-, Alkoxyalkyl, Acyloxyalkyl-, Acytylalkyl-Rest, vorzugsweise Wasserstoff oder Methyletherrest ist;
R2 Wasserstoff, ein Alkyl-, Alkoxy-, Halogen-, Acyl oder Etheralkylaldehyd-, Hydroxy-, Hydroxyalkyl-, Alkoxyalkyl, Acyloxyalkyl-, Acetylalkylrest, vorzugsweise Wasserstoff oder Halogenrest ist;
R3 eine Amin- oder Hydroxyalkylgruppe ist, die mit einer Ferrocengruppe durch eine kovalente Bindung verbunden ist, und die Salze dieser Komplexe.

2. Komplex nach Anspruch 1, dadurch gekennzeichnet, dass er eine der folgenden Strukturen besitzt: 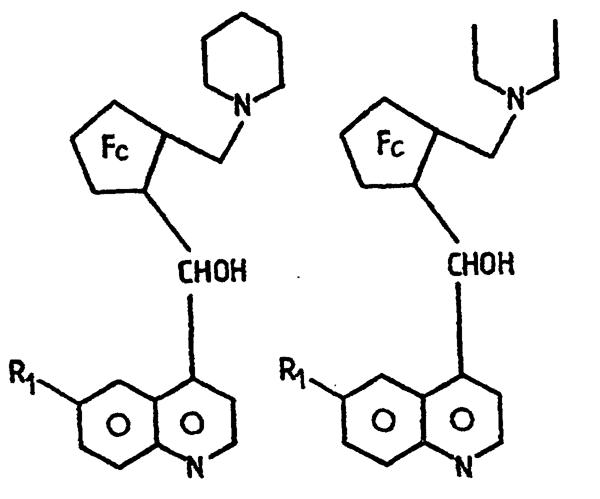 worin
R1 die vorher angegebene Bedeutung besitzt.

3. Komplex nach Anspruch 1, dadurch gekennzeichnet, dass er die folgende Struktur besitzt: 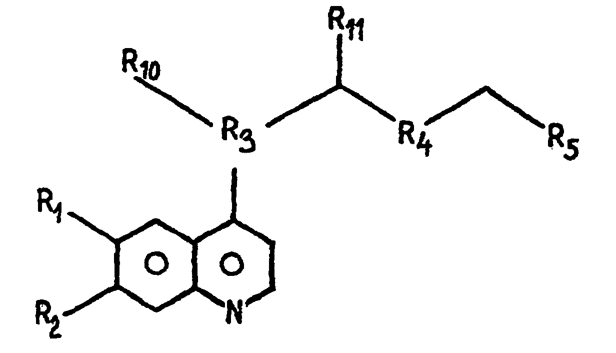 worin
R1, R2 und R3 die im Anspruch 1 angegebene Bedeutung besitzen;
R4 eine Alkyl-, Alkoxy-, Acyl-, Hydroxyalkyl-, Acyloxyalkyl-, Acetylalkyl- oder Ferrocengruppe ist;
R5 eine Amingruppe ist, welche gegebenenfalls durch eine oder mehrere Alkylgruppen oder durch eine oder mehrere Alkylferrocengruppen substitutiert ist;
R10 Wasserstoff, ein Alkyl- oder ein Alkylferrocen ist; und R11 Wasserstoff oder ein Alkylrest ist.

4. Komplex nach Anspruch 3, dadurch gekennzeichnet, dass er eine der folgenden Strukturen besitzt: 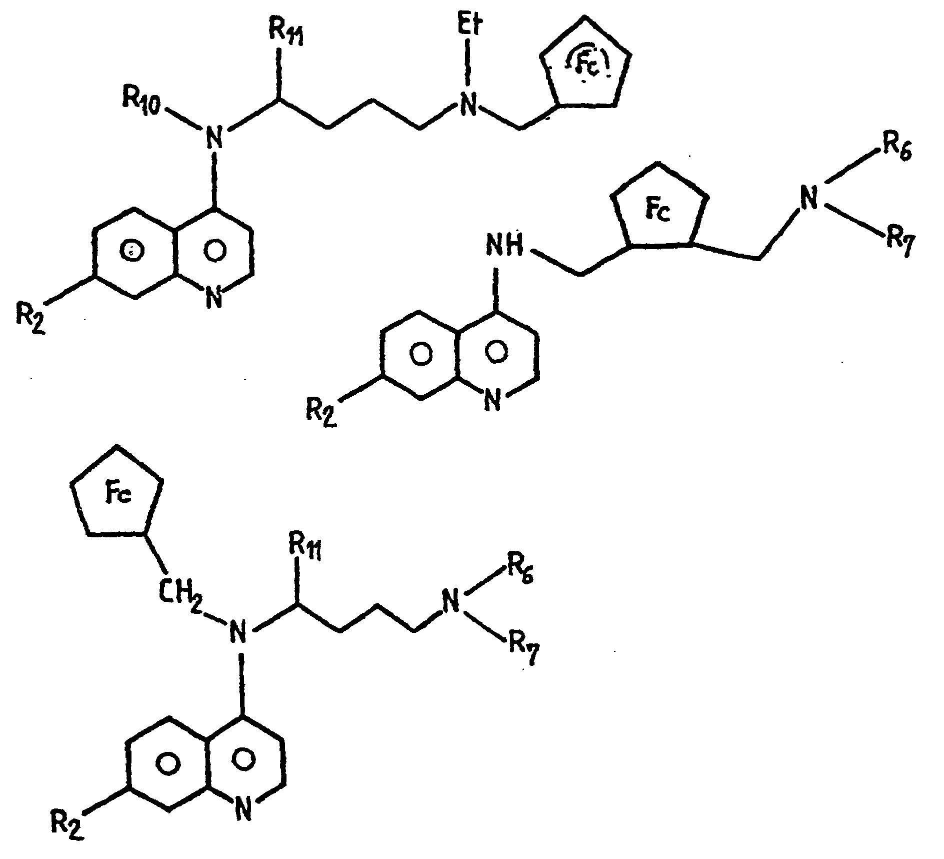 worin
R2, R10 und R11 die vorher angegebene Bedeutung besitzen; und R6 und R7 gleich oder verschieden sind und aus Wasserstoff, einem Alkylferrocen-, Ferrocen-, Alkyl-Rest, insbesondere einem Methyl- oder Ethylrest ausgewählt sind, aber auch die Gruppe 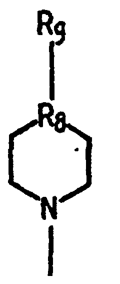 worin R8 -CH₂- oder ein Heteroatom ist und R9 Wasserstoff, ein Alkyl-, Alkoxy-, Acyl-, Halogen- oder Etheralkylaldehyd-, Hydroxy-, Hydroxyalkyl-, Alkoxyalkyl-, Acyloxyalkyl-, Acetylalkylrest ist, um vorzugsweise di - Piperidin- 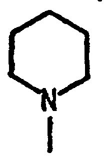 Morpholin- 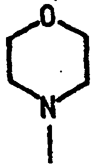 oder Piperazin- 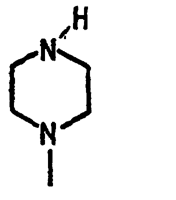 Gruppe ist.

5. Komplex nach Anspruch 1, dadurch gekennzeichnet, dass er die folgende Struktur besitzt: 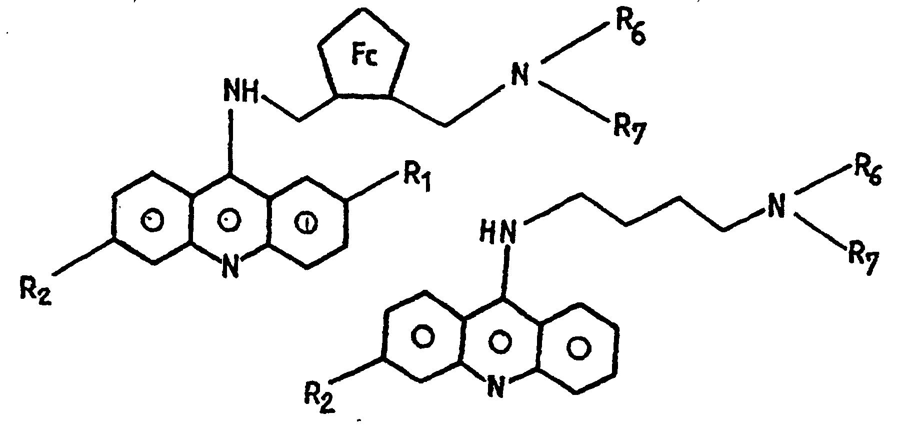 worin
R1 und R2 die im Anspruch 1 angegebene Bedeutung besitzen; und R6 und R7 die im Anspruch 4 angegebene Bedeutung besitzen.

6. Komplex nach Anspruch 1, dadurch gekennzeichnet, dass er die folgende Struktur besitzt: 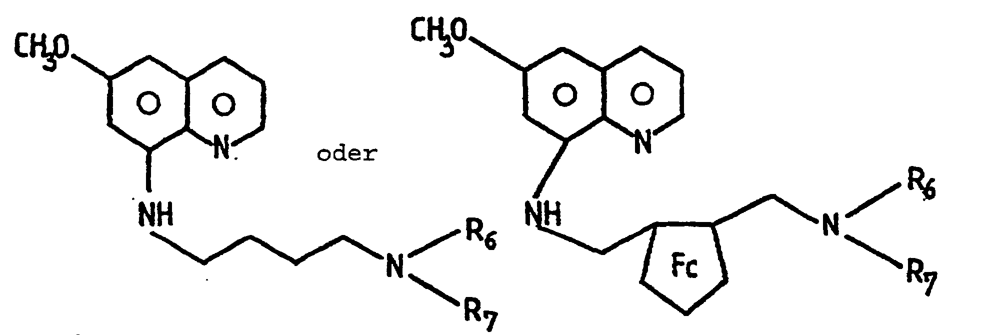 worin
R6 und R7 die im Anspruch 4 angegebene Bedeutung besitzen.

7. Komplex nach einem der Ansprüche 1 bis 6, dadurch gekennzeichnet, dass die Salze Tartrat- oder Ditartratsalze sind.

8. Zusammensetzung zur Behandlung von Malaria, dadurch gekennzeichnet, dass sie einen in den Ansprüchen 1 bis 7 definierten Organometallkomplex in Kombination mit einem pharmazeutisch annehmbaren Träger umfasst.

9. Verwendung einer Verbindung nach den Ansprüchen 1 bis 7 zur Herstellung eines Medikamentes zur Behandlung von Malaria.

## Claims

1. Organometallic iron complex comprising at least one of the basic structural elements characteristic of a molecule possessing antimalarial properties as well as one or more ferrocenyl groups, wherein the structural element characteristic of a molecule possessing antimalarial properties includes: 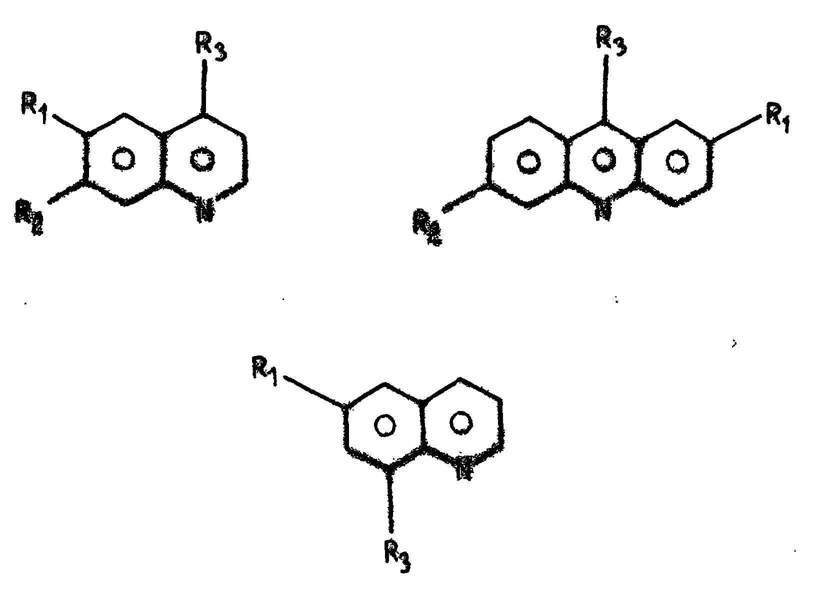 in which
R₁ is hydrogen, alkyl, alkoxy, acyl, halogen or etheralkyl aldehyde, hydroxyl, hydroxyalkyl, alkoxyalkyl, acyloxyalkyl, acetylalkyl, preferably hydrogen or ethermethyl;
R₂ is hydrogen, alkyl, alkoxy, halogen, acyl or etheralkyl aldehyde, hydroxyl, hydroxyalkyl, alkoxyalkyl, acyloxyalkyl, acetylalkyl, preferably hydrogen or halogen; and
R₃ is an amine or hydroxyalkyl group which can be linked to a ferrocene group by means of a covalent bond, and the salts of the said complexes.

2. Complex according to claim 1, characterized in that it possesses one of the following structures: 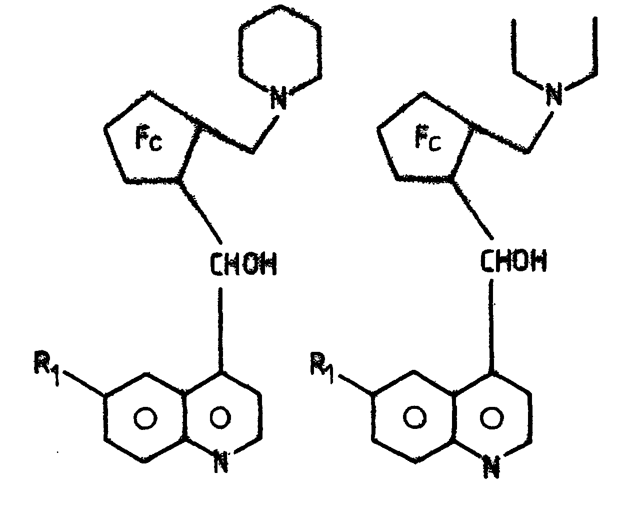 in which
R₁ is as previously defined.

3. Complex according to claim 1, characterized in that it possesses the following structure: 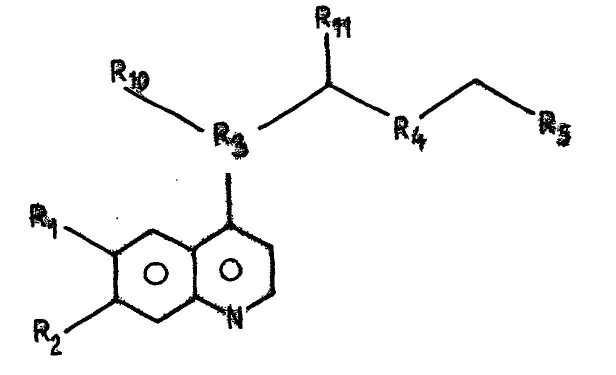 in which
R₁, R₂ and R₃ are as defined in claim 1;
R₄ is an alkyl, alkoxy, acyl, hydroxyalkyl, acyloxyalkyl, acetylalkyl or ferrocene group;
R₅ is an amine group, non substituted or substituted with one or more alkyl groups or with one or more alkylferrocene groups;
R₁₀ is hydrogen, alkyl or an alkyl-ferrocene group; and
R₁₁ is hydrogen or alkyl.

4. Complex according to Claim 3, characterized in that it possesses one of the following structures: 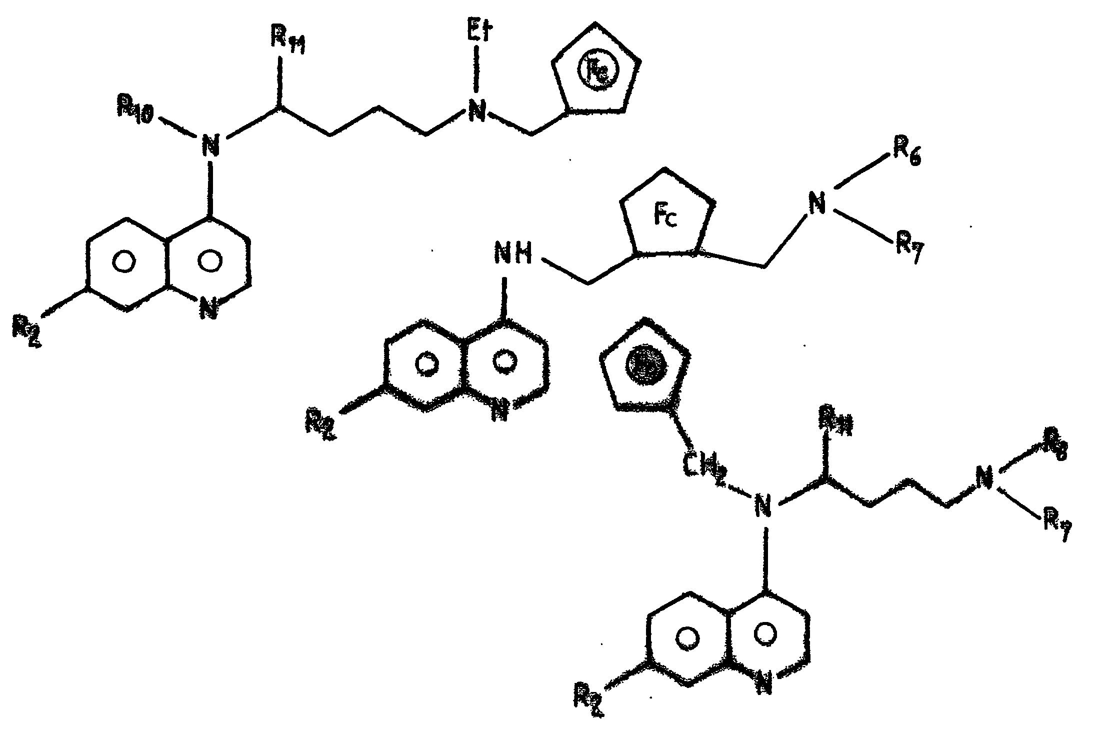 in which
R₂, R₁₀ and R₁₁ are as defined above; and R₆ and R₇ are identical or different and are selected from hydrogen, alkyl-ferrocene, ferrocene, alkyl, particularly methyl or ethyl, but also the group 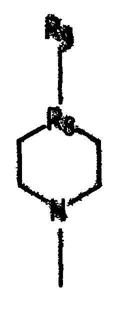 where R₈ is a -CH₂- or an heteroatom, and R₉ is hydrogen, alkyl, alkoxy, acyl, halogen or etheralkyl aldehyde, hydroxyl, hydroxyalkyl, alkoxyalkyl, acyloxyalkyl, acetylalkyl, to form, preferably, the groups piperidino 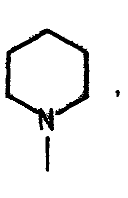 morpholino 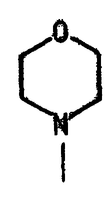 or piperazino

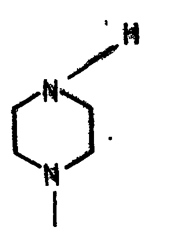

5. Complex according to claim 1, characterized in that it possesses the following structure: 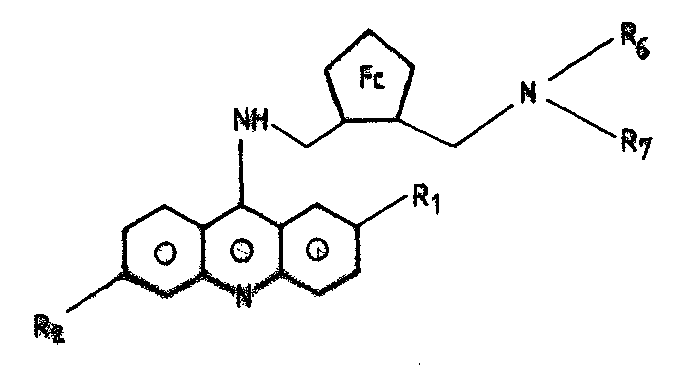 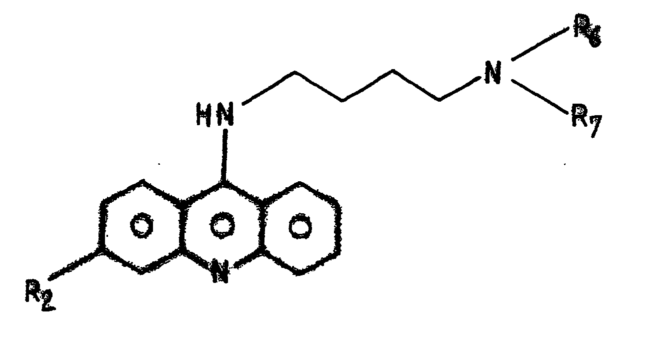 n which
R₁ and R₂ are as defined in claim 1; and
R₆ and R₇ are as defined in Claim 4.

6. Complex according to claim 1, characterized in that it possesses the following structure: 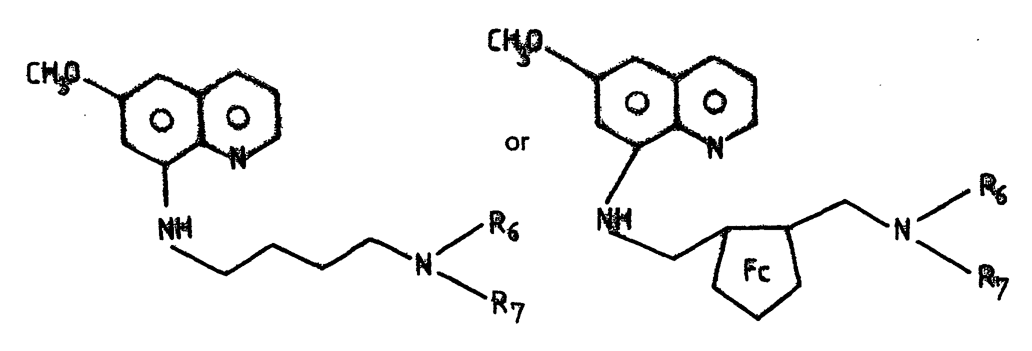 in which
R₆ and R₇ are as defined in Claim 4.

7. Complex according to any of claims 1 to 6, characterized in that the salts are the tartrate or ditartrate salts.

8. Composition for the treatment of malaria, characterized in that it comprises an organometallic complex as defined in claims 1 to 7, in combination with an acceptable pharmaceutical excipient.

9. Use of a compound according to any one of claims 1 to 7 for the preparation of a medicament intended for the treatment of malaria.
